# EUROPEAN PATENT APPLICATION

(11) **EP 4 799 570 A1**
(43) Date of publication of application: **02.09.2026**
(21) Application number: 24902613.9
(22) Date of filing: 02.12.2024
(51) Int. Cl.: A61B 5/287

(54) **HIGH-DENSITY MAPPING CATHETER**

(30) Priority: 12.12.2023 CN 202311704515
(71) Applicant: Shanghai Microport Ep Medtech Co., Ltd., Shanghai 201318 (CN)
(72) Inventor: ZENG, Jin, Shanghai 201318 (CN); TAO, Runhan, Shanghai 201318 (CN); ZHAO, Li, Shanghai 201318 (CN); LIN, Hang, Shanghai 201318 (CN); LIANG, Bo, Shanghai 201318 (CN)
(74) Representative: Marks & Clerk LLP
(86) International application number: PCT/CN2024/136052
(87) International publication number: WO 2025/124192

(57) **Abstract**

The present invention provides a high-density mapping catheter including a catheter body and a distal mapping part. The distal mapping part includes a plurality of mapping branches and a far-field electrode provided on one of the mapping branches. A proximal end of each mapping branch is fixedly connected to a distal end of the catheter body and each mapping branch comprises a first connecting portion and a spine portion. At least two ring electrodes are sequentially arranged on the spine portion along a lengthwise direction thereof. The spine portion of each mapping branch is connected to the distal end of the catheter body through the respective first connecting portion. The far-field electrode is provided on the first connecting portion of one of the mapping branches and is configured to be allowed to come into contact with blood, but not with any tissue, to obtain far-field signals. Thus, in the high-density mapping catheter of the present invention, the arrangement of the far-field electrode does not rely on the presence of a central saline irrigation lumen in the catheter any longer, allowing for optimization of conventional irrigation designs for saline irrigation on both sides.

## Description

### TECHNICAL FIELD

The present invention relates to the field of medical devices and, in particular, to a high-density mapping catheter.

### BACKGROUND

Atrial fibrillation (AF), one of the most cardiac arrhythmias encountered in clinical practice and poses a major serious challenge to the global cardiovascular field in the 21st century. Catheter ablation is one of the currently available effective therapies for AF treatment, and mapping catheters provide an important tool for physicians to identify sites of disease origin and plan ablation strategies. Among these, high-density mapping catheters enable signal mapping of the entire cardiac chamber, reducing the mapping time.

In conventional high-density mapping catheters, reference electrodes are arranged outside the central saline tube of the catheter to collect intracardiac far-field signals. This design requires the catheter to have a central saline irrigation pathway, which imposes significant constraints on the design of the catheter's saline irrigation pathway.

### SUMMARY OF THE INVENTION

It is an object of the present invention to provide a high-density mapping catheter, which overcomes the prior-art problem of significant limitations on the design of an irrigation pathway of the catheter arising from the arrangement of reference electrodes externally around a central saline irrigation tube.

To this end, the present invention provides a high-density mapping catheter comprising a catheter body and a distal mapping part.

The distal mapping part comprises a plurality of mapping branches and a far-field electrode provided on one of the mapping branches. A proximal end of each mapping branch is fixedly connected to a distal end of the catheter body.

Each mapping branch comprises a first connecting portion and a spine portion. At least two ring electrodes are sequentially arranged on each spine portion along a lengthwise direction thereof, wherein the ring electrode is configured to be brought into contact with tissue to obtain an electrocardiogram (ECG) signal, and wherein the spine portion of each mapping branch is connected to the distal end of the catheter body through a corresponding first connecting portion.

The far-field electrode is provided on the first connecting portion of the mapping branch and is configured to be allowed to come into contact with blood, but not with any tissue, to obtain a far-field signal.

Optionally, in the high-density mapping catheter, the plurality of mapping branches diverge toward a distal end so that the distal mapping part comprises a claw-like structure, or wherein the plurality of mapping branches first diverge toward a distal end and then converge to form a three-dimensional mesh-like structure.

Optionally, in the high-density mapping catheter, the plurality of mapping branches are connected in pairs to form at least two loop members, and wherein distal ends of the loop members remain relatively fixed.

Optionally, in the high-density mapping catheter, under a first side view, the distal mapping part comprises a mesh, wherein one loop member is located on an inner side and each remaining loop member surrounds the loop member that is located on the inner side, and
under a second side view, the loop member located on the inner side and other loop member(s) are located on different two planes, and wherein the two planes intersect on an extension line of an axis of the catheter body,
wherein the second side view is perpendicular to the first side view.

Optionally, in the high-density mapping catheter, distal ends of the plurality of mapping branches are all coupled through a distal rod to form a closed two-dimensional mesh-like structure.

Optionally, in the high-density mapping catheter, the far-field electrode may be provided on the first connecting portion of the mapping branch that is closer to the inner side.

Optionally, in the high-density mapping catheter, the first connecting portion may be provided with insulating members at opposite ends of the far-field electrode, wherein a first height difference is provided between a surface of the insulating member and a surface of the first connecting portion and a second height difference is provided between a surface of the far-field electrode and the surface of the first connecting portion, and the first height difference is greater than the second height difference.

Optionally, in the high-density mapping catheter, a third height difference may be provided between the surface of the insulating member and the surface of the far-field electrode, and wherein the third height difference is less than 0.5 mm.

Optionally, in the high-density mapping catheter, the insulating members may be formed by applying an insulating adhesive to the opposite ends of the far-field electrode and then curing the adhesive.

Optionally, in the high-density mapping catheter, a distance between the far-field electrode and the distal end of the catheter body is not greater than 5 mm.

Optionally, in the high-density mapping catheter, a distal end of the catheter body comprises a retainer, wherein a proximal end of each mapping branch is retained by the retainer, wherein the retainer comprises a saline irrigation pathway, and wherein the saline irrigation pathway comprises a main channel and two branch channels in communication with a distal end of the main channel.

Optionally, in the high-density mapping catheter, the retainer comprises two rows of retention bores configured to retain the proximal ends of the mapping branches located on the two different planes, wherein an inlet of the main channel is located on one side of one of the rows of retention bores away from the other row of retention bores, and wherein outlets of the two branch channels are located on sides of the two rows of retention bores that are away from each other.

Optionally, in the high-density mapping catheter, each mapping branch may comprise an internal support and an insulating layer covering the internal support, a cross-section of the internal support of at least the spine portion is a rectangle, and wherein a long side of the rectangle is oriented in a direction in which the mapping branch comes into contact with the tissue.

In summary, the present invention provides a high-density mapping catheter including a catheter body and a distal mapping part. The distal mapping part includes a plurality of mapping branches and a far-field electrode provided on one of the mapping branches. Each mapping branch is secured at its proximal end to a distal end of the catheter body and includes a first connecting portion and a spine portion. At least two ring electrodes are sequentially arranged on the spine portion along a lengthwise direction thereof. The ring electrodes are configured to be brought into contact with tissue to obtain ECG signals. The spine portion of each mapping branch is connected to the distal end of the catheter body through the respective first connecting portion. The far-field electrode is provided on the first connecting portion of the mapping branch and configured to be allowed to come into contact with blood, but not with any tissue, to obtain far-field signals. Thus, in the high-density mapping catheter of the present invention, the far-field electrode is spaced apart from the distal end of the catheter body, and therefore its arrangement does not rely on the presence of a central saline irrigation pathway in the catheter any longer, allowing for optimization of conventional irrigation designs and achieving saline irrigation on both sides.

### BRIEF DESCRIPTION OF THE DRAWINGS

Those of ordinary skill in the art will understand that the following drawings are presented to enable a better understanding of the present invention and not intended to limit the scope thereof in any sense, in which:
Fig. 1 shows a schematic overall view of a high-density mapping catheter according to an embodiment of the present invention;
Fig. 2 schematically illustrates a distal end of a high-density mapping catheter according to an embodiment of the present invention, when viewed from a first side;
Fig. 3 schematically illustrates an arrangement of insulating members according to an embodiment of the present invention;
Fig. 4 schematically illustrates a distal end of a high-density mapping catheter according to an embodiment of the present invention, when viewed from a second side;
Fig. 5 schematically illustrates a distal mapping part according to an embodiment of the present invention, which includes two loop members;
Fig. 6 schematically illustrates a distal mapping part according to an embodiment of the present invention, which includes three loop members;
Fig. 7 shows a schematic partial view of a distal end of a high-density mapping catheter according to an embodiment of the present invention;
Fig. 8 shows a schematic cross-sectional view taken along line B-B of Fig. 7;
Fig. 9 shows a schematic cross-sectional view taken along line A-A of Fig. 7;
Figs. 10 to 14 schematically illustrate distal end of other high-density mapping catheters, in each of which a far-field electrode is arranged in the same way as in embodiments of the present invention;
Fig. 15 shows a schematic cross-sectional view taken along line C-C of Fig. 2;
Fig. 16 shows a schematic cross-sectional view of a spine portion according to an embodiment of the present invention; and
Fig. 17 shows a schematic cross-sectional view of another spine portion according to an embodiment of the present invention.

### List of Reference Numerals

1 distal mapping part; 2 steerable section; 3 main section; 4 handle;
10 mapping branch;
101 first connecting portion; 102 spine portion; 103 second connecting portion; 104 ring electrode; 105 far-field electrode; 106 insulating member; 107 retainer; 108 coupling member;
201, 202 branch channel; 203 main channel;
301 first loop member; 302 second loop member; 303 third loop member; 304 fourth loop member; 305 fifth loop member;
401, 402, 403, 404, 405, 406 retention bore;
501 ring-shaped member; 502 first linear member; 503 second linear member;
11 internal support; 12 insulating layer;
110 electrode lead.

### DETAILED DESCRIPTION

The present invention will be described in greater detail below with reference to the accompanying drawings, which illustrate specific embodiments thereof. From the following description, advantages and features of the present invention will become more apparent. Note that the figures are provided in a very simplified form not necessarily drawn to exact scale for the only purpose of facilitating easy and clear description of the embodiments disclosed herein. In addition, the illustrated structures are usually part of their real-world counterparts. In particular, as the figures tend to have distinct emphases, they are sometimes drawn to different scales. Further, it is to be understood that, as used herein, the terms "first", "second", "third" and the like are only meant to distinguish various components, elements, steps, etc. from each other and are not intended to indicate logical or sequential orderings thereof, unless otherwise indicated or specified. Accordingly, defining an item with "first", "second", "third" or the like is an explicit or implicit indication of the presence of one or at least two such items.

As used herein, the terms "proximal end" and "distal end" describe relative orientations, positions and directions of components of a medical device and actions taken thereon, as viewed by a physician operating the device. While not wishing to be limiting, "proximal end" usually refers to an end of the medical device closer to the physician, and "distal end" to an end thereof that enters the body of a patient first, during normal operation of the device.

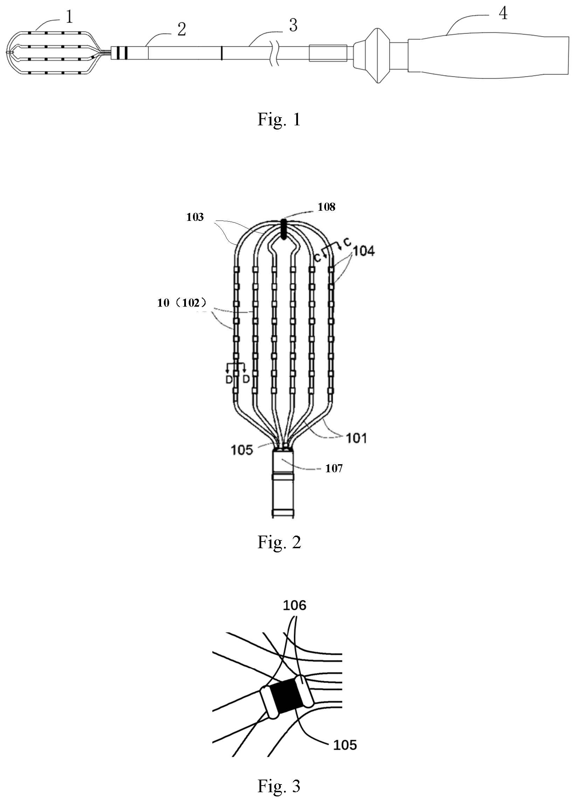

Referring to Fig. 1, embodiments of the present invention provide a high-density mapping catheter including a catheter body and a distal mapping part 1. The catheter body may be of any suitable structure well known in the art and typically includes a handle 4, a main section 3 and a steerable section 2, which are sequentially joined along the direction from a proximal end to a distal end. The structure of the catheter body will be further described below.

Referring to Fig. 2, the distal mapping part includes a plurality of mapping branches 10 and a far-field electrode 105 provided on one of the mapping branches. Each of the mapping branches 10 is fixedly connected, at a proximal end thereof, to a distal end of the catheter body and includes a first connecting portion 101 and a spine portion 102, which is connected to the distal end of the catheter body through the first connecting portion 101. At least two ring electrodes 104 are sequentially arranged along a lengthwise direction of the spine portion. The ring electrodes 104 are configured to be brought into contact with tissue to obtain electrocardiogram (ECG) signals. The spine portion 102 of each mapping branch 10 is connected to the distal end of the catheter body through the respective first connecting portion 101, and the far-field electrode 105 is provided on the first connecting portion 101 of the mapping branch 10. The far-field electrode 105 is configured to be allowed to come into contact with blood, but not with any tissue, to obtain far-field signals. Preferably, a distance between the far-field electrode 105 and the distal end of the catheter body is not greater than 5 mm. That is, the far-field electrode 105 is disposed in the neighborhood of roots of the mapping branches 10.

When used in an electrophysiology ablation, the high-density mapping catheter may be inserted into a patient's heart via a femoral vein puncture so as to bring the soft distal mapping part into contact with cardiac tissue to obtain intracardiac electrical signals. However, signals obtained by the ring electrodes in the distal mapping part may include other interfering signals, such as far-field signals from blood or other tissue. In the high-density mapping catheter of the present embodiment, the distal mapping part 1 includes both the ring electrodes, which can be brought into contact with tissue to collect electrical signal information in multiple directions, and the far-field electrode 105, which is arranged in the vicinity of the proximal ends of the mapping branches 10 without contacting any tissue to collect ECG signals conducted by blood from regions away from said contacted tissue.

In addition, according to embodiments of the present invention, since the far-field electrode 105 is spaced apart from the distal end of the catheter body in the high-density mapping catheter, its arrangement does not rely on the presence of a central saline irrigation pathway in the catheter any longer, allowing for optimization of conventional irrigation designs for saline irrigation on both sides.

Preferably, as shown in Fig. 3, the first connecting portion 101 is provided with insulating members 106 at opposite ends of the far-field electrode 105. There are a first height difference between a surface of the insulating member 106 and a surface of the first connecting portion 101 and a second height difference between a surface of the far-field electrode 105 and the surface of the first connecting portion 101. The first height difference is greater than the second height difference, leading to the far-field electrode 105 and the insulating members 106 on its opposite sides together forming a concave structure with higher ends and a lower middle, which can reduce the probability of the far-field electrode 105 coming into contact with tissue. Preferably, there is a height difference between the surface of the insulating member 106 and the surface of the far-field electrode 105, which is less than 0.5 mm.

In some embodiments, the insulating member 106 may be formed by applying an adhesive to the opposite ends of the far-field electrode 105 and then curing the adhesive. These embodiments provide ease of operation. In some alternative embodiments, the insulating members 106 may be ring-shaped insulating material that is sleeved over the first connecting portion 101.

As described above, the arrangement of the far-field electrode 105 according to this embodiment allows for optimization of conventional irrigation designs for achieving saline irrigation on both sides. In one preferred implementation achieving saline irrigation on both sides, as shown in Fig. 2, the catheter body may include a retainer 107 at its distal end, the mapping branches 10 pass through the retainer 107, and the retainer 107 comprises a saline irrigation pathway including a main channel and two branch channels in communication with a distal end of the main channel. The main channel and the two branch channels are all eccentrically arranged, and outlets of the two branch channels are located at opposite ends of an axis of the retainer 107.

In order to achieve the main channel and the two branch channels that are arranged in the retainer 107, the retainer 107 is preferred to be an assembled structure, in which one of the branch channels has a lateral branch formed by transverse assembling of the assembling structure. With this arrangement, by inserting a single saline tube in the main channel, saline can flow into both branch channels, achieving irrigation on both sides without increasing a diameter of the catheter. Thus, better irrigation performance can be obtained.

In one optional embodiment, the mapping branches 10 in the distal mapping part are connected in pairs to form at least two loop members, the distal ends of the loop members remain relatively fixed. In this optional embodiment, under a first side view, i.e., as shown in the side view of Fig. 2, the distal mapping part generally appears like a mesh, in which some of the mapping branches 10 are located on inner side closer to an extension line of the axis of the catheter body, while some outer mapping branches 10 are located on outer side farther away from the extension line of the axis of the catheter body. Compared with the mapping branches 10 located on the inner side, the mapping branches 10 located on the outer side are more prone to deformation, and hence more likely to come into contact with tissue. In view of this, it is preferred to provide the far-field electrode 105 on the first connecting portion 101 of one of the mapping branches 10 closer to the inner side, in order to reduce the probability of the far-field electrode 105 coming into contact with any tissue.

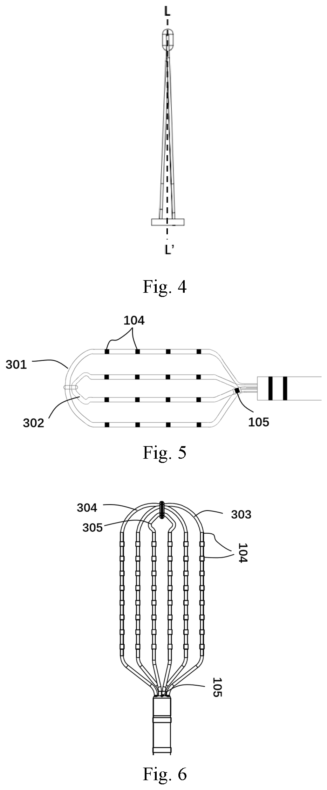

In order to allow the ring electrodes 104 to come into contact with tissue in various orientations, in this embodiment, the distal mapping part 1 may be further designed so that, under the first side view, one of the loop members is located on the inner side, and each remaining loop member is arranged surrounding the inner loop member, and that, under a second side view, the inner loop member and the other loop members are distributed on two different planes, and the two planes intersect on the extension line of the axis of the catheter body. As such, under the second side view, as shown in Fig. 4, the distal mapping part 1 in the high-density mapping catheter appears inverted V-shaped. The second side view and the first side view are two side views perpendicular to each other.

With continued reference to Fig. 1, in order for easier connection to be achieved, in this embodiment, each first connecting portion 101 may be curved. In addition to the spine portions 102 and the first connecting portions 101, each loop member may further include a second connecting portion 103, which may be curved in shape and connected at its opposite ends to distal ends of the two spine portions 102 of the loop member. The second connecting portions 103 in the loop members may be all fastened using a coupling member 108.

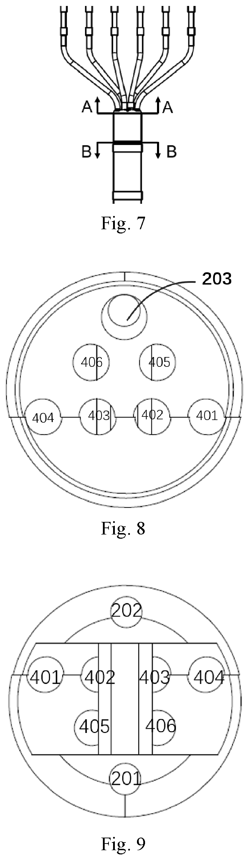

In the above-described embodiment, the eccentric arrangement of the main channel can avoid it from interfering with the retention of the mapping branches 10 in the retainer 107. Specifically, in order to render the inner mapping branches 10 not coplanar with the outer mapping branches 10, as shown in Figs. 8 and 9, the retainer 107 comprises two rows of retention bores for retaining the inner mapping branches 10 and the outer mapping branches 10. One of the rows of retention bores is configured to retain proximal ends of the outer mapping branches 10, and the other row of retention bores is configured to retain proximal ends of the inner mapping branches 10. In order to avoid interference, an inlet of the main channel 203 is provided on one side of one of the rows of retention bores that is away from the other row of retention bores. In order to achieve irrigation on both sides, the outlets of the two branch channels 201, 202 are arranged on sides of the two rows of retention bores that are away from each other.

In the foregoing embodiment, there may be one inner loop member and one, two, three or more outer loop members. In one example, as shown in Fig. 5, the catheter distal mapping part includes two loop members, namely, a first loop member 301 and a second loop member 302. Under the first side view, the first loop member 301 is located on the inner side, and the second loop member 302 is located on the outer side. The distal ends of the first loop member 301 and the second loop member 302 are fastened by the coupling member 108, and no intersection ion is present between them. In this case, each of the two rows of retention bores in the retainer 107 contains two retention bores for retaining the proximal ends of the two branches of the first 301 or second 302 loop member.

In another example, as shown in Fig. 6, the catheter distal mapping part includes three loops, namely, a third loop member 303, a fourth loop member 304 and a fifth loop member 305. The third loop member 303 and the fourth loop member 304 together make up an outer mapping member. The third loop member 303 and the fourth loop member 304 may overlap and intersect each other at a single point. Alternatively, the third loop member 303 and the fourth loop member 304 may be arranged vertically one above the other at their distal ends, but not intersect each other. The fifth loop member 305 is disposed on the inner side of the entirety made up of the third loop member 303 and the fourth loop member, without intersecting either of them. In this case, one of the rows of retention bores in the retainer 107 contains four retention bores for retaining the respective proximal ends of the two loop members located on the outer side. Four branches of the two loop members may be arranged corresponding to the four retention bores indicated at 401, 402, 403 and 404 in Figs. 8 and 9. As shown, the four branches may be interposed, or the two branches in one of the loop members may be located between the two branches in the other loop member. The other row of retention bores contains two retention bores for retaining the proximal ends of the two branches in the loop member located on the inner side. The two branches in the loop member may be arranged corresponding to the two retention bores indicated at 405 and 406 in Figs. 8 and 9.

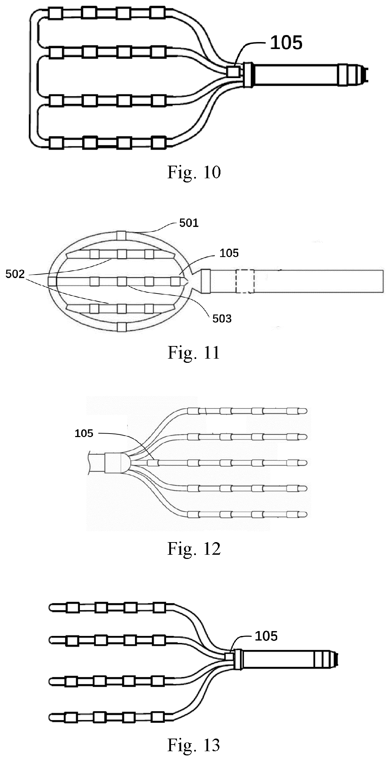

The arrangement of the far-field electrode 105 according to embodiments of the present invention is also suitable for use in distal catheters with other types of distal mapping structures. For example, as shown in Fig. 10, distal ends of multiple mapping branches 10 may be connected by a single distal rod to form a closed two-dimensional mesh-like structure. As another example, as schematically illustrated in Fig. 11, there is another catheter with a mesh-like mapping structure. The catheter distal mapping part includes a ring-shaped member 501, first linear members 502 and a second linear member 503. A proximal end of the second linear member 503 (located in the middle) is attached to a distal end of a catheter body, and a distal end thereof is attached to the ring-shaped member 501. The two first linear members 502 are disposed on opposite sides of the second linear member 503, and the proximal and distal ends of each of the first linear members 502 is attached to the ring-shaped member 501. Each of the ring-shaped member 501, the first linear members 502 and the second linear member 503 constitutes the mapping branch 10. It will be understood that, in both cases, there is a mapping branch 10 closer to the inner side, and a far-field electrode 105 may be provided on a first connecting portion 101 of the mapping branch 10 that is closer to the inner side.

In addition, as shown in Figs. 12 and 13, in distally non-closed high-density mapping catheters, a far-field electrode 105 may also be provided on the root of a central mapping branch 10 or on the root of a mapping branch 10 close to the center, i.e., around a proximal end of corresponding mapping branch 10.

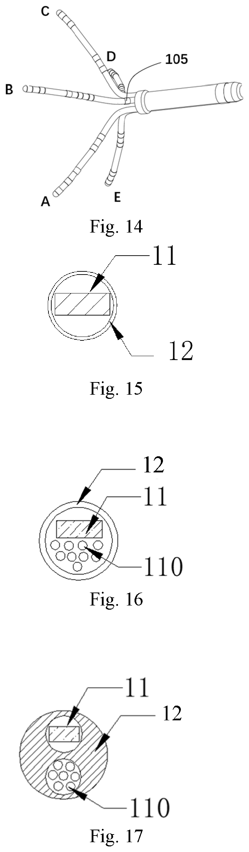

For multiple mapping branches that diverge toward the distal end such that the distal mapping part forms a claw-like structure as shown in Fig. 14, or for multiple mapping branches that first diverge toward the distal end and then converge to form a three-dimensional mesh-like structure of a high-density mapping catheter (not shown), according to the above design approach for the far-field electrode 105, there can be multiple options. The far-field electrode 105 can be arranged at the root of any branch, which can also avoid contact with tissue.

Further, in this embodiment, as shown in Fig. 15, each mapping branch 10 may include an internal support 11 and an insulating layer 12 covering the internal support 11. Preferably, the internal support 11 in at least the spine portion 102 has a rectangular cross-section with long sides oriented in a direction in which the mapping branch comes into contact with tissue, i.e., in the direction indicated by line C-C of Fig. 2. The internal support 11 is made of a metal material with desirable supporting properties, such as a nickel alloy. The cross-section of the internal support 11 is configured as a rectangular structure, which facilitates the contact between the electrode-carrying portion and the tissue. The insulating layer 12 may be made of polyamide or polyurethane and have an outer diameter less than 3F, which can facilitate use of the catheter with an 8F or 8.5F sheath.

Figs. 16 and 17 schematically illustrate optional internal structures for the spine portion 102. As shown in Fig. 16, the spine portion 102 may comprise a single lumen, the internal support 11 and electrode leads 110 are located in the lumen. Alternatively, as shown in Fig. 17, inside the spine portion 102, two lumens are separated by the insulating layer 12, and the internal support 11 and the electrode leads 110 are located in different lumens.

Furthermore, in this embodiment, the steerable section 2 may use tubular section with multiple lumens, preferably four lumens, including a lumen configured for passage of a saline tube therethrough, a lumen for receiving leads therein, and two symmetrical lumens serving as pull wire lumens configured for the passage of a pull wire therethrough. The main section 3 is an elongate tubular section containing braid wires and configured for connection with both the multi-lumen tubular section and the handle. The braid wires can provide functions of, among others things, providing support for the tubular body, preventing deformation and enabling proportional torque transmission. The handle 4 may be provided at its proximal end with a pigtail socket, to which leads in the catheter can be connected. The main section 3 may be coupled to the handle 4 via a handle knob, which is tied to the steerable section 2 by a pull wire in order to enable steering control of the steerable section 2. Alternatively, a steering member may be provided on one side of a wall of the handle 4 and tie to the steerable section 2 by a pull wire in order to enable steering control of the steerable section 2.

In summary, embodiments of the present invention provide a high-density mapping catheter including a catheter body and a distal mapping part. The distal mapping part includes a plurality of mapping branches and a far-field electrode provided on one of the mapping branches. Each mapping branch is fixedly connected at its proximal end to a distal end of the catheter body and includes a first connecting portion and a spine portion. At least two ring electrodes are sequentially arranged on the spine portion along a lengthwise direction thereof. The ring electrodes are configured to be brought into contact with tissue to obtain electrocardiogram (ECG) signals. The spine portion of each mapping branch is connected to the distal end of the catheter body through the respective first connecting portion. The far-field electrode is provided on the first connecting portion of one mapping branch and configured to be allowed to come into contact with blood, but not with any tissue, to obtain far-field signals. In the high-density mapping catheter of the present invention, the far-field electrode is arranged outside the distal end of the catheter body, and therefore its arrangement does not rely on the presence of a central saline irrigation pathway in the catheter any longer, allowing for optimization of conventional irrigation designs and achieving saline irrigation on both sides.

The description presented above is merely that of some preferred embodiments of the present invention and is not intended to limit the scope thereof in any sense. Any and all changes and modifications made by those of ordinary skill in the art based on the above teachings fall within the scope of the invention.

## Claims

1. A high-density mapping catheter, comprising a catheter body and a distal mapping part,
wherein the distal mapping part comprises a plurality of mapping branches and a far-field electrode provided on one of the mapping branches, wherein a proximal end of each mapping branch is fixedly connected to a distal end of the catheter body;
wherein each mapping branch comprises a first connecting portion and a spine portion, wherein at least two ring electrodes are sequentially arranged on each spine portion along a lengthwise direction thereof, wherein the ring electrode is configured to be brought into contact with tissue to obtain an electrocardiogram (ECG) signal, and wherein the spine portion of each mapping branch is connected to the distal end of the catheter body through a corresponding first connecting portion,
wherein the far-field electrode is provided on the first connecting portion of one of the mapping branches and is configured to be allowed to come into contact with blood, but not with tissue, to obtain a far-field signal.

2. The high-density mapping catheter of claim 1, wherein the plurality of mapping branches diverge toward a distal end so that the distal mapping part comprises a claw-like structure, or wherein the plurality of mapping branches first diverge toward a distal end and then converge to form a three-dimensional mesh-like structure.

3. The high-density mapping catheter of claim 1, wherein the plurality of mapping branches are connected in pairs to form at least two loop members, and wherein distal ends of the loop members remain relatively fixed.

4. The high-density mapping catheter of claim 3, wherein under a first side view, the distal mapping part comprises a mesh, wherein one loop member is located on an inner side and each remaining loop member surrounds the loop member that is located on the inner side, and
wherein under a second side view, the loop member located on the inner side and other loop member(s) are located on different two planes, and wherein the two planes intersect on an extension line of an axis of the catheter body,
wherein the second side view is perpendicular to the first side view.

5. The high-density mapping catheter of claim 1, wherein distal ends of the plurality of mapping branches are all coupled through a distal rod to form a closed two-dimensional mesh-like structure.

6. The high-density mapping catheter of claim 3 or 5, wherein the far-field electrode is provided on the first connecting portion of the mapping branch that is closer to the inner side.

7. The high-density mapping catheter of claim 1, wherein the first connecting portion is provided with insulating members at opposite ends of the far-field electrode, wherein a first height difference is provided between a surface of the insulating member and a surface of the first connecting portion, wherein a second height difference is provided between a surface of the far-field electrode and the surface of the first connecting portion, and wherein the first height difference is greater than the second height difference.

8. The high-density mapping catheter of claim 7, wherein a third height difference is provided between the surface of the insulating member and the surface of the far-field electrode, and wherein the third height difference is less than 0.5 mm.

9. The high-density mapping catheter of claim 7, wherein the insulating member is formed by applying an insulating adhesive to the opposite ends of the far-field electrode and then curing the insulating adhesive.

10. The high-density mapping catheter of claim 1, wherein a distance between the far-field electrode and the distal end of the catheter body is not greater than 5 mm.

11. The high-density mapping catheter of claim 1, wherein a distal end of the catheter body comprises a retainer, wherein a proximal end of each mapping branch is retained by the retainer, wherein the retainer comprises a saline irrigation pathway, and wherein the saline irrigation pathway comprises a main channel and two branch channels in communication with a distal end of the main channel.

12. The high-density mapping catheter of claim 11, wherein the retainer comprises two rows of retention bores configured to retain the proximal ends of the mapping branches located on the two different planes, wherein an inlet of the main channel is located on one side of one of the rows of retention bores away from the other row of retention bores, and wherein outlets of the two branch channels are located on sides of the two rows of retention bores that are away from each other.

13. The high-density mapping catheter of claim 1, wherein each mapping branch comprises an internal support and an insulating layer covering the internal support, wherein a cross-section of the internal support of at least the spine portion is a rectangle, and wherein a long side of the rectangle is oriented in a direction in which the mapping branch comes into contact with the tissue.
